(19) Europäisches Patentamt / European Patent Office / Office européen des brevets

(11) **EP 3 294 777 B1**

## (12) EUROPEAN PATENT SPECIFICATION

(45) Date of publication and mention
of the grant of the patent:
**26.06.2019 Bulletin 2019/26**

(21) Application number: **16726232.8**

(22) Date of filing: **10.05.2016**

(51) Int Cl.:
**C08B 13/00** (2006.01)     **A61K 47/38** (2006.01)

(86) International application number:
**PCT/US2016/031591**

(87) International publication number:
**WO 2016/186897 (24.11.2016 Gazette 2016/47)**

(54) **PROCESS FOR PRODUCING ESTERIFIED CELLULOSE ETHERS OF VERY HIGH MOLECULAR WEIGHT AND LOW VISCOSITY**

HERSTELLUNG ESTERIFIZIERTER CELLULOSEETHER MIT HOHEM MOELKULARGEWICHT UND GERINGER VISKOSITÄT

PROCESSUS DE PRODUCTION DE CELLULOSE ETHERS ESTÉRIFIÉS DE POIDS MOLÉCULAIRE TRÈS ÉLEVÉ ET UNE FAIBLE VISCOSITÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.05.2015 US 201562162087 P**

(43) Date of publication of application:
**21.03.2018 Bulletin 2018/12**

(73) Proprietor: **Dow Global Technologies LLC
Midland, MI 48674 (US)**

(72) Inventor: **PETERMANN, Oliver
29699 Bomlitz (DE)**

(74) Representative: **f & e patent
Braunsberger Feld 29
51429 Bergisch Gladbach (DE)**

(56) References cited:
EP-A1- 0 018 547        EP-A1- 2 810 660
EP-A2- 0 018 605        WO-A1-2011/159626
WO-A1-2014/031448        GB-A- 2 006 217
US-A1- 2014 249 235

• Wanwilai Darunkaisorn ET AL: "HPMC Matrix Granule Formation: Selection of Suitable Granulating Fluid", Thai Pharmaceutical and Health Science Journal Thai Pharm Health Sci J, vol. 44, no. 29, 1 January 2009 (2009-01-01), pages 29-45, XP055502947,
• Xuefei Cao ET AL: "Rapid Synthesis of Cellulose Esters by Transesterification of Cellulose with Vinyl Esters under the Catalysis of NaOH or KOH in DMSO", Journal of Agricultural and Food Chemistry, vol. 61, no. 10, 27 February 2013 (2013-02-27), pages 2489-2495, XP055503022, US ISSN: 0021-8561, DOI: 10.1021/jf3055104

**Description**

FIELD

The present invention relates to an improved process for preparing an esterified cellulose ether.

INTRODUCTION

Esters of cellulose ethers, their uses and processes for preparing them are generally known in the art. Known methods of producing cellulose ether-esters include the reaction of a cellulose ether with an aliphatic monocarboxylic acid anhydride and a dicarboxylic acid anhydride, for example as described in U.S Patent Nos. 4,226,981 and 4,365,060.

Various known esters of cellulose ethers are useful as enteric polymers for pharmaceutical dosage forms, such as hydroxypropyl methylcellulose acetate succinate (HPMCAS). Enteric polymers are those that are resistant to dissolution in the acidic environment of the stomach. Dosage forms coated with such polymers protect the drug from inactivation or degradation in the acidic environment or prevent irritation of the stomach by the drug. US Patent No. 4,365,060 discloses enterosoluble capsules which are said to have excellent enterosolubility behavior.

European Patent Application EP 0 219 426 discloses a process for producing an enteric-soluble acidic dicarboxylic acid ester of a cellulose ether wherein (a) a cellulose ether having hydroxypropoxyl groups as the ether-forming groups, of which a 2% by weight aqueous solution has a viscosity of at least 5 centipoise at 20 °C, is reacted with (b) a dicarboxylic acid anhydride or a mixture thereof with an anhydride of an aliphatic monocarboxylic acid in the presence of (c) a combination of an alkali metal acetate and acetic acid. EP 0 219 426 shows that the acidic dicarboxylic acid esters produced from cellulose ethers which have a viscosity of at least 6 centipoise provided an enterosoluble film-coating material on tablets which had resistance against a simulated gastric juice. When comparative acidic dicarboxylic acid esters were produced from cellulose ethers having a viscosity of only 3 centipoise, a substantial number of tablets disintegrated in the simulated gastric juice. Acidic dicarboxylic acid esters produced from cellulose ethers of higher viscosity have a higher molecular weight than those produced from cellulose ethers of lower viscosity when comparable process and recipe parameters for producing the acidic dicarboxylic acid esters are applied.

It is known in the prior art, e.g. as published in International Patent Application WO 2005/115330, that HPMCAS is also useful to increase the bioavailability of poorly water-soluble drugs. This is of great importance as nearly 70% of new drug candidates are low water soluble compounds. As a general rule, poorly water soluble drugs possess low bioavailability. The HPMCAS is aimed at reducing the crystallinity of the drug, thereby minimizing the activation energy necessary for the dissolution of the drug, as well as establishing hydrophilic conditions around the drug molecules, thereby improving the solubility of the drug itself to increase its bioavailability, i.e., its *in vivo* absorption by an individual upon ingestion. In view of the great usefulness of HPMCAS, much research has been spent on modifying HPMCAS and processes for preparing them.

International Patent Applications WO 2005/115330 and WO 2011/159626 disclose HPMCAS polymers with a specific combination of substitution levels.

International Patent Application WO 2014/133885 discloses HPMCAS of a specific substitution pattern of succinoyl groups and acetyl groups. The HPMCAS is produced by reacting acetic acid and succinic acid in separate steps with hydroxypropyl methylcellulose.

It is recognized in the art that the substitution levels and the substitution pattern of HPMCAS have an impact on its ability to increase the bioavailability of poorly water-soluble drugs. It is believed that the molecular weight of an esterified cellulose ether like HPMCAS also has an important impact on its ability to increase the bioavailability of poorly water-soluble drugs. Edgar et al., Cellulose (2007), 14:49-64 mention a study on microparticle formation of theophylline with two CABs (cellulose acetate butyrates) of similar composition, differing substantially only in molecular weight. The release of theophylline was slowed dramatically by higher polymer molecular weight, speeded dramatically by lower particle size, and reduced substantially by particle formation from a higher viscosity solution.

International Patent Applications WO2014/031447 and WO2014/031448 disclose methods of controlling the molecular weight of HPMCAS. WO2014/031447 discloses that the molecular weight of HPMCAS increases with decreasing molar ratio [aliphatic carboxylic acid / anhydroglucose units of cellulose ether]. WO2014/031448 discloses that the molecular weight of HPMCAS increases with increasing molar ratio [alkali metal carboxylate / anhydroglucose units of cellulose ether]. The aliphatic carboxylic acid and the alkali metal carboxylate are used as reaction diluent and reaction catalyst, respectively.

While esterified cellulose ethers of high molecular weight are very desirable for good resistance against a gastric juice, known esterified cellulose ethers of high molecular weight exhibit a high viscosity when they are dissolved at a high concentration in an organic solvent, such as a concentration of 7 - 10 weight percent. A high viscosity reduces their efficiency in coating processes. In coating processes high concentrations of the esterified cellulose ether in an organic solvent are desired to minimize the amount of solvent that has to be subsequently removed. On the other hand, the viscosity of the solution should be low to facilitate spraying of the solution on the dosage forms, such as tablets, to be coated.

International Patent Application Dow case WO2014/137779 discloses esterified cellulose ethers like HPMCAS which have a high molecular weight and a reasonably low viscosity in acetone and a process for producing them.

In view of the great utility and importance of esterified cellulose ethers like HPMCAS it is an object of the present invention to enrich the art and to find new ways of producing esterified cellulose ethers. It is a preferred object of the present invention to find new ways of producing esterified cellulose ethers which have a high molecular weight but which can still be efficiently used in spray-drying and coating processes.

## SUMMARY

Surprisingly, it has been found that the ratio of molecular weight and viscosity in acetone of an esterified cellulose ether like HPMCAS can be optimized, i.e., that its viscosity can be decreased without significantly decreasing its molecular weight and/or its molecular weight can be increased without significantly increasing its viscosity, by feeding a reaction diluent at different stages to the reaction mixture at the production of the esterified cellulose ether.

Accordingly, the present invention relates to a process for producing an esterified cellulose ether which comprises the stages of

i) preparing a reaction mixture comprising a cellulose ether, an aliphatic monocarboxylic acid anhydride, a dicarboxylic acid anhydride, and a reaction diluent and heating the reaction mixture to a temperature of from 60°C to 110 °C prior to, during or after mixing the components of the reaction mixture to conduct an esterification reaction, and
ii) before the esterification reaction is completed, adding an additional amount of reaction diluent continuously or in one or more portions to the reaction mixture and allowing the esterification reaction to further proceed.

## DESCRIPTION OF EMBODIMENTS

The cellulose ether used as a starting material in the process of the present invention has a cellulose backbone having β-1,4 glycosidically bound D-glucopyranose repeating units, designated as anhydroglucose units in the context of this invention. The cellulose ether preferably is an alkyl cellulose, hydroxyalkyl cellulose or hydroxyalkyl alkylcellulose. This means that in the cellulose ether utilized in the process of the present invention, at least a part of the hydroxyl groups of the anhydroglucose units are substituted by alkoxyl groups or hydroxyalkoxyl groups or a combination of alkoxyl and hydroxyalkoxyl groups. The hydroxyalkoxyl groups are typically hydroxymethoxyl, hydroxyethoxyl and/or hydroxypropoxyl groups. Hydroxyethoxyl and/or hydroxypropoxyl groups are preferred. Typically one or two kinds of hydroxyalkoxyl groups are present in the cellulose ether. Preferably a single kind of hydroxyalkoxyl group, more preferably hydroxypropoxyl, is present. The alkoxyl groups are typically methoxyl, ethoxyl and/or propoxyl groups. Methoxyl groups are preferred.

Illustrative of the above-defined cellulose ethers are alkylcelluloses, such as methylcellulose, ethylcellulose, and propylcellulose; hydroxyalkylcelluloses, such as hydroxyethylcellulose, hydroxypropylcellulose, and hydroxybutylcellulose; and hydroxyalkyl alkylcelluloses, such as hydroxyethyl methylcellulose, hydroxymethyl ethylcellulose, ethyl hydroxyethylcellulose, hydroxypropyl methylcellulose, hydroxypropyl ethylcellulose, hydroxybutyl methylcellulose, and hydroxybutyl ethylcellulose; and those having two or more hydroxyalkyl groups, such as hydroxyethylhydroxypropyl methylcellulose. Most preferably, the cellulose ether is a hydroxyalkyl methylcellulose, such as hydroxypropyl methylcellulose.

The degree of the substitution of hydroxyl groups of the anhydroglucose units by hydroxyalkoxyl groups is expressed by the molar substitution of hydroxyalkoxyl groups, the MS(hydroxyalkoxyl). The MS(hydroxyalkoxyl) is the average number of moles of hydroxyalkoxyl groups per anhydroglucose unit in the cellulose ether. It is to be understood that during the hydroxyalkylation reaction the hydroxyl group of a hydroxyalkoxyl group bound to the cellulose backbone can be further etherified by an alkylation agent, e.g. a methylation agent, and/or a hydroxyalkylation agent. Multiple subsequent hydroxyalkylation etherification reactions with respect to the same carbon atom position of an anhydroglucose unit yields a side chain, wherein multiple hydroxyalkoxyl groups are covalently bound to each other by ether bonds, each side chain as a whole forming a hydroxyalkoxyl substituent to the cellulose backbone.

The term "hydroxyalkoxyl groups" thus has to be interpreted in the context of the MS(hydroxyalkoxyl) as referring to the hydroxyalkoxyl groups as the constituting units of hydroxyalkoxyl substituents, which either comprise a single hydroxyalkoxyl group or a side chain as outlined above, wherein two or more hydroxyalkoxy units are covalently bound to each other by ether bonding. Within this definition it is not important whether the terminal hydroxyl group of a hydroxyalkoxyl substituent is further alkylated, e.g. methylated, or not; both alkylated and non-alkylated hydroxyalkoxyl substituents are included for the determination of MS(hydroxyalkoxyl). The cellulose ether utilized in the process of the invention generally has a molar substitution of hydroxyalkoxyl groups in the range 0.05 to 1.00, preferably 0.08 to 0.90, more preferably 0.12 to 0.70, most preferably 0.15 to 0.60, and particularly 0.20 to 0.40.

The average number of hydroxyl groups substituted by alkoxyl groups, such as methoxyl groups, per anhydroglucose unit, is designated as the degree of substitution of alkoxyl groups, DS(alkoxyl). In the above-given definition of DS, the term "hydroxyl groups substituted by alkoxyl groups" is to be construed within the present invention to include not only alkylated hydroxyl groups directly bound to the carbon atoms of the cellulose backbone, but also alkylated hydroxyl groups of hydroxyalkoxyl substituents bound to the cellulose backbone. The cellulose ethers utilized in the process of the invention generally have a DS(alkoxyl) in the range of 1.0 to 2.5, preferably from 1.1 to 2.4 , more preferably from 1.2 to 2.2 most preferably from 1.6 to 2.05, and particularly from 1.7 to 2.05.

The degree of substitution of alkoxyl groups and the molar substitution of hydroxyalkoxyl groups can be determined by Zeisel cleavage of the cellulose ether with hydrogen iodide and subsequent quantitative gas chromatographic analysis (G. Bartelmus and R. Ketterer, Z. Anal. Chem., 286 (1977) 161-190). Most preferably the cellulose ether utilized in the process of the invention is hydroxypropyl methylcellulose having a DS(methoxyl) within the ranges indicated above for DS(alkoxyl) and an MS(hydroxypropoxyl) within the ranges indicated above for MS(hydroxyalkoxyl).

The cellulose ether used as a starting material in the process of the present invention generally has a viscosity of up to 200 mPa·s, preferably up to 100 mPa·s, more preferably up to 50 mPa·s, and most preferably up to 5 mPa·s, measured as a 2 weight-% aqueous solution at 20 °C according to ASTM D2363 - 79 (Reapproved 2006). Generally their viscosity is at least 1.2 mPa·s, typically at least 1.8 mPa·s, even more typically at least 2.4 mPa·s, and most typically at least 2.8 mPa·s, measured as a 2 weight-% aqueous solution at 20 °C. Cellulose ethers of such viscosity can be obtained by subjecting a cellulose ether of higher viscosity to a partial depolymerization process. Partial depolymerization processes are well known in the art and described, for example, in European Patent Applications EP 1 141 029; EP 0 210 917; EP 1 423 433; and US Patent No. 4,316,982. Alternatively, partial depolymerization can be achieved during the production of the cellulose ethers, for example by the presence of oxygen or an oxidizing agent.

The molar number of anhydroglucose units of the cellulose ether utilized in the process of the present invention can be determined from the weight of the cellulose ether used as a starting material, by calculating the average molecular weight of the substituted anhydroglucose units from the DS(alkoxyl) and MS(hydroxyalkoxyl).

In stage i) of the process of the present invention a reaction mixture is prepared which comprises a cellulose ether, an aliphatic monocarboxylic acid anhydride, a dicarboxylic acid anhydride and a reaction diluent. In one embodiment of the invention a reaction mixture is prepared which additionally comprises a reaction catalyst, such as an alkali metal carboxylate. The reaction mixture is heated to a temperature of from 60°C to 110 °C prior to, during or after mixing the components of the reaction mixture to conduct an esterification reaction.

In one embodiment of stage i) of the invention, the entire amounts of cellulose ether, aliphatic monocarboxylic acid anhydride, dicarboxylic acid anhydride and reaction catalyst, such as alkali metal carboxylate, utilized in the process of the invention and a portion of the reaction diluent utilized in the process are combined to provide the reaction mixture. In this embodiment the mixture is preferably heated during or, more preferably, after mixing the components of the reaction mixture.

In another embodiment of the invention, stage i) of the invention is divided into several steps. Preferably stage i) of the process comprises the steps of iA) dissolving or dispersing a cellulose ether and a first amount of a reaction catalyst, such as an alkali metal carboxylate, in a reaction diluent, iB) heating the obtained mixture to a temperature of 60 °C to 110 °C before, during or after adding an aliphatic monocarboxylic acid anhydride and a dicarboxylic acid anhydride to the mixture obtained in step iA), and iC) adding a second amount of a reaction catalyst, such as an alkali metal carboxylate. A preferred alkali metal carboxylate is sodium acetate or potassium acetate. In step iA) first the cellulose ether or first the reaction catalyst, such as alkali metal carboxylate, or both simultaneously can be dissolved or dispersed in the reaction diluent. Only a portion of the total amount of reaction catalyst, such as alkali metal carboxylate, that is added to the reaction mixture in stage i) of the process is added in step iA). Preferably only 15 to 35 percent, more preferably only 20 to 30 percent of the total amount of reaction catalyst is added in step iA).

Preferably the esterification reaction is allowed to proceed in the reaction mixture of step iB) before a second amount of a reaction catalyst, such as an alkali metal carboxylate, is added to the reaction mixture. Typically the esterification reaction is allowed to further proceed in the reaction mixture of step iC) after the addition of a second amount of reaction catalyst. However, after step iC) the esterification reaction should not be completed yet.

Before completing the esterification reaction in stage i) of the reaction, stage i) being optionally divided into steps iA), iB) and iC), in stage ii) of the process an additional amount of reaction diluent is added continuously or in one or more portions to the reaction mixture and the esterification reaction is allowed to further proceed.

Preferred starting materials, their amounts and reaction conditions utilized in stage i) of the reaction, wherein stage i) is optionally divided into steps iA), iB) and iC), and in stage ii) of the reaction are described below.

The reaction diluent preferably is an aliphatic carboxylic acid, such as acetic acid, propionic acid, or butyric acid. The reaction diluent can comprise minor amounts of other solvents or diluents which are liquid at room temperature and do not react with the cellulose ether, such as halogenated $C_1$-$C_3$ derivatives, such as dichloro methane, or dichloro methyl ether, but the amount of the aliphatic carboxylic acid generally is more than 50 percent, preferably at least 75 percent, and more preferably at least 90 percent, based on the total weight of the reaction diluent. Most preferably the reaction diluent essentially consists of an aliphatic carboxylic acid. The reaction mixture in stage i) generally comprises from 100 to 2,000 parts by weight, preferably from 100 to 1,000 parts by weight, and more preferably from 100 to 250 parts by weight of a reaction diluent per 100 parts by weight of the cellulose ether. When an aliphatic carboxylic acid is used as a reaction diluent, the molar ratio [aliphatic carboxylic acid / anhydroglucose units of cellulose ether] in stage i) generally is from [4.0 / 1.0] to [11.5 / 1.0], preferably from [5.0 / 1.0] to [11.0 / 1.0], more preferably from [6.0 / 1.0] to [9.0 / 1.0], and most preferably from [6.2 / 1.0] to [7.7 / 1.0].

A preferred aliphatic monocarboxylic acid anhydride is acetic anhydride, butyric anhydride or propionic anhydride. The molar ratio between the anhydride of aliphatic monocarboxylic acid and the anhydroglucose units of the cellulose ether generally is 0.1 / 1 or more, preferably 0.3 / 1 or more, and more preferably 0.5 / 1 or more, most preferably 1.0 / 1 or more, and particularly 2.0 / 1 or more. The molar ratio between the aliphatic monocarboxylic acid anhydride and the anhydroglucose units of the cellulose ether generally is 10 / 1 or less, preferably 8 / 1 or less, more preferably 6 / 1 or less, most preferably 4 / 1 or less, and particularly 3.5 / 1 or less.

A preferred dicarboxylic acid anhydride is succinic anhydride, maleic anhydride or phthalic anhydride. Succinic anhydride or phthalic anhydride is more preferred. Succinic anhydride is the most preferred dicarboxylic acid anhydride. The molar ratio between the anhydride of a dicarboxylic acid and the anhydroglucose units of the cellulose ether generally is at least 0.01 / 1, preferably at least 0.04 / 1, more preferably at least 0.2 / 1, and most preferably at least 0.6 / 1. The molar ratio between the anhydride of a dicarboxylic acid and the anhydroglucose units of cellulose ether generally is up to 2.5 / 1, preferably up to 1.5 / 1, and more preferably up to 1 /v1. In stage i) of the reaction process, the molar ratio of (a) aliphatic carboxylic acid to (b) dicarboxylic acid anhydride, (a)/(b), is generally up to 12 / 1, typically from 7.0 / 1 to 12.0 / 1.

More preferably the cellulose ether is esterified with succinic anhydride or phthalic anhydride in combination with an aliphatic monocarboxylic acid anhydride selected from the group consisting of acetic anhydride, butyric anhydride and propionic anhydride. Most preferably, hydroxypropyl methylcellulose is reacted with succinic anhydride and acetic anhydride to produce hydroxypropyl methyl cellulose acetate succinate.

In stage i) of the reaction, wherein stage i) is optionally divided into steps iA), iB) and iC), the reaction mixture is heated to a temperature of from 60 °C to 110 °C, preferably from 70 to 100 °C prior to, during or after mixing the components of the reaction mixture. In a preferred embodiment the cellulose ether, the amount of reaction diluent utilized in stage i) and the entire amount or a portion of the esterification catalyst as described above are first heated to and kept in the above-mentioned temperature range from 5 to 90 minutes, preferably from 10 to 30 minutes under agitation, followed by addition of the anhydride of a dicarboxylic acid and the anhydride of an aliphatic monocarboxylic acid.

When stage i) is divided into steps iA), iB) and iC), in step iC) a second amount of reaction catalyst, such as an alkali metal carboxylate, is added to the reaction mixture. Preferably from 65 to 85 percent, more preferably from 70 to 80 percent of the total amount of added reaction catalyst is added in step iC). The second amount of reaction catalyst is preferably added at a time period of 10 to 90 minutes, more preferably from 15 to 60 minutes, after the last addition of the anhydrides.

The reaction time for the esterification reaction in stage i), calculated from the addition of the last component of the reaction mixture in step i), typically is from 30 minutes to 6 hours, more typically from 45 minutes to 4 hours, and most typically from 1 to 3 hours. The reaction time in stage i) generally is at least 20%, preferably at least 25%, and more preferably at least 30% of the total reaction time in stages i) and ii) of the process of the present invention. The reaction time in stage i) generally is up to 80%, preferably up to 75%, and more preferably up to 70% of the total reaction time in stages i) and ii).

Before the esterification reaction is completed, in stage ii) an additional amount of reaction diluent is added continuously or in one or more portions to the reaction mixture and the esterification reaction is allowed to further proceed. More preferably the additional amount of reaction diluent is added in 1 or 2 portions, most preferably in 1 portion. Preferably the added amount of a reaction diluent in stage ii) is from 20 to 200 parts by weight, and more preferably from 40 to 100 parts by weight of reaction diluent per 100 parts by weight of the cellulose ether that has been added to the reaction mixture in stage i). When an aliphatic carboxylic acid is used as a reaction diluent, the molar ratio [aliphatic carboxylic acid / anhydroglucose units of cellulose ether] added in stage ii) generally is from [0.5 / 1.0] to [7.0 / 1.0], preferably from [0.7 / 1.0] to [6.0 / 1.0], more preferably from [5.0 / 1.0] to [9.0 / 1.0], and most preferably from [4.0 / 1.0] to [7.7 / 1.0]. Preferably from 50 to 90 percent, and more preferably from 60 to 85 percent of the reaction diluent is added in stage i) of the process and preferably from 10 to 50 percent, and more preferably from 15 to 40 percent of the reaction diluent is added in stage ii) of the process of the present invention, based on the total weight of the reaction diluent in the process.

Preferably the additional amount of a reaction diluent is added to the reaction mixture when more than 20%, more preferably when more than 30%, of the entire reaction time in the process of the present invention has passed. Preferably the additional amount of reaction diluent is added to the reaction mixture at a time when less than 80%, more preferably when less than 70%, of the entire reaction time in the process has passed. After addition of the last amount of reaction diluent in step ii), the reaction mixture is generally kept in the temperature range from 60 °C to 110 °C, preferably from 70 to 100 °C, for 10 minutes to 6 hours, more typically from 20 minutes to 5 hours, and most typically from 30 minutes to 4 hours.

After completion of the esterification reaction, the reaction product can be precipitated from the reaction mixture in a known manner, for example by contacting the reaction mixture with a large volume of water, such as described in U.S. Patent No. 4,226,981, International Patent Application WO 2005/115330 or European Patent Application EP 0 219 426. In a preferred embodiment of the invention the reaction product is precipitated from the reaction mixture as described in International Patent Application PCT/US13/030394, published as WO2013/148154, to produce an esterified cellulose ether in the form of a powder.

By the process of the present invention preferably esterified cellulose ethers are produced which comprise groups of the formula - C(O) - R - COOH, wherein R is a divalent aliphatic or aromatic hydrocarbon group, such as -C(O)-CH$_2$-CH$_2$-COOH, -C(O)-CH=CH-COOH or -C(O)-C$_6$H$_4$-COOH, and monovalent acyl groups, such as acetyl, propionyl, or butyryl, such as n-butyryl or i-butyryl. Specific examples of esterified cellulose ethers are hydroxypropyl methyl cellulose acetate phthalate (HPMCAP), hydroxypropyl methyl cellulose acetate maleate (HPMCAM) or hydroxypropyl methylcellulose acetate succinate (HPMCAS); hydroxypropyl cellulose acetate succinate (HPCAS), hydroxybutyl methyl cellulose propionate succinate (HBMCPrS), hydroxyethyl hydroxypropyl cellulose propionate succinate (HEHPCPrS); or methyl cellulose acetate succinate (MCAS). Hydroxypropyl methylcellulose acetate succinate (HPMCAS) is the most preferred esterified cellulose ether.

The esterified cellulose ethers produced according to the process of the present invention generally have a degree of substitution of aliphatic monovalent acyl groups, such as acetyl, propionyl, or butyryl groups, of at least 0.05, preferably at least 0.10, and more preferably at least 0.25. The esterified cellulose ethers generally have a degree of substitution of aliphatic monovalent acyl groups of up to 1.5, preferably up to 1.0, and more preferably up to 0.6. The esterified cellulose ethers generally have a degree of substitution of groups of formula -C(O) - R - COOH, such as succinoyl, of at least 0.01, preferably at least 0.05, and most preferably at least 0.10. The esterified cellulose ethers generally have a degree of substitution of groups of formula -C(O) - R - COOH of up to 1.3, preferably up to 0.8, and more preferably up to 0.5.

The total degree of ester substitution is generally at least 0.2, preferably at least 0.5, more preferably at least 0.7, and most preferably at least 0.85. The total degree of ester substitution is generally not more than 1.5, preferably not more than 1.4, more preferably not more than 1.3 and most preferably not more than 1.2.

The content of the acetate and succinate ester groups is determined according to "Hypromellose Acetate Succinate, United States Pharmacopia and National Formulary, NF 29, pp. 1548-1550". Reported values are corrected for volatiles (determined as described in section "loss on drying" in the above HPMCAS monograph). The method may be used in analogue manner to determine the content of propionyl, butyryl, phthalyl and other ester groups. The content of ether groups in the esterified cellulose ether is determined in the same manner as described for "Hypromellose", United States Pharmacopeia and National Formulary, USP 35, pp 3467-3469. The contents of ether and ester groups obtained by the above analyses are converted to DS and MS values of individual substituents according to the formulas below. The formulas may be used in analogue manner to determine the DS and MS of substituents of other cellulose ether esters.

$$\% \text{ cellulose backbone}$$
$$= 100 - \left( \%\text{MeO} * \frac{\text{M(OCH}_3) - \text{M(OH)}}{\text{M(OCH}_3)} \right)$$
$$- \left( \%\text{HPO} * \frac{\text{M(OCH}_2\text{CH(OH)CH}_3) - \text{M(OH)}}{\text{M(OCH}_2\text{CH(OH)CH}_3)} \right)$$
$$- \left( \%\text{Acetyl} * \frac{\text{M(COCH}_3) - \text{M(H)}}{\text{M(COCH}_3)} \right)$$
$$- \left( \%\text{Succinoyl} * \frac{\text{M(COC}_2\text{H}_4\text{COOH)} - \text{M(H)}}{\text{M(COC}_2\text{H}_4\text{COOH)}} \right)$$

$$\text{DS(Me)} = \frac{\frac{\%\text{MeO}}{\text{M(OCH}_3)}}{\frac{\%\text{cellulose backbone}}{\text{M(AGU)}}} \qquad \text{MS(HP)} = \frac{\frac{\%\text{HPO}}{\text{M(HPO)}}}{\frac{\%\text{cellulose backbone}}{\text{M(AGU)}}}$$

$$DS(Acetyl) = \frac{\frac{\%Acetyl}{M(Acetyl)}}{\frac{\%cellulose\ backbone}{M(AGU)}} \qquad DS(Succinoyl) = \frac{\frac{\%Succinoyl}{M(Succinoyl)}}{\frac{\%cellulose\ backbone}{M(AGU)}}$$

$M(MeO) = M(OCH_3) = 31.03$ Da $M(HPO) = M(OCH_2CH(OH)CH_3) = 75.09$ Da $M(Acetyl) = M(COCH_3) = 43.04$ Da $M(Succinoyl) = M(COC_2H_4COOH) = 101.08$ Da $M(AGU) = 162.14$ Da $M(OH) = 17.008$ Da $M(H) = 1.008$ Da

By convention, the weight percent is an average weight percentage based on the total weight of the cellulose repeat unit, including all substituents. The content of the methoxyl group is reported based on the mass of the methoxyl group (i.e., $-OCH_3$). The content of the hydroxyalkoxyl group is reported based on the mass of the hydroxyalkoxyl group (i.e., -O-alkylene-OH); such as hydroxypropoxyl (i.e., $-O-CH_2CH(CH_3)-OH$). The content of the aliphatic monovalent acyl group is reported based on the mass of $-C(O) - R_1$ wherein $R_1$ is a monovalent aliphatic group, such as acetyl ($-C(O)-CH_3$). The content of the group of formula -C(O) - R - COOH is reported based on the mass of this group, such as the mass of succinoyl groups (i.e., $-C(O)-CH_2-CH_2COOH$).

Esterified cellulose ethers are produced by the process of the present invention which have a surprisingly high weight average molecular weight $M_w$ at a surprisingly low viscosity in acetone. Esterified cellulose ethers of typically up to 500,000 Dalton, more typically up to 480,000 Dalton, and most typically up to 460,000 Dalton are produced by the process of the present invention. Generally they have a weight average molecular weight $M_w$ of at least 100,000 Dalton, more typically at least 200,000 Dalton, even more typically at least 300,000 Dalton. $M_w$ and $M_n$ are measured according to Journal of Pharmaceutical and Biomedical Analysis 56 (2011) 743 using a mixture of 40 parts by volume of acetonitrile and 60 parts by volume of aqueous buffer containing 50 mM $NaH_2PO_4$ and 0.1 M $NaNO_3$ as mobile phase. The mobile phase is adjusted to a pH of 8.0. The measurement of $M_w$ and $M_n$ $M_z$ is described in more details in the Examples.

The esterified cellulose ethers produced by the process of the present invention are soluble in acetone and have a reasonably low viscosity. Generally the esterified cellulose ethers have a viscosity of up to 200 mPa·s, preferably up to 100 mPa·s, more preferably up to 90 mPa·s, and most preferably up to 80 mPa·s, measured as a 10 wt.-% solution of the esterified cellulose ether in acetone at 20 °C. The esterified cellulose ethers typically have a viscosity of 10 mPa·s or more, typically of 20 mPa·s or more, more typically of 30 mPa·s or more, and most typically of 40 mPa·s or more, measured as a 10 wt.-% solution of the esterified cellulose ether in acetone at 20 °C.

Some embodiments of the invention will now be described in detail in the following Examples.

EXAMPLES

Unless otherwise mentioned, all parts and percentages are by weight. In the Examples the following test procedures are used.

Viscosity of Hydroxypropyl Methyl Cellulose Acetate Succinate (HPMCAS)

The 10 wt.-% solution of HPMCAS in acetone was prepared by mixing 10.0 g HPMCAS, based on its dry weight, with 90.0 g of acetone under vigorous stirring at room temperature. The mixture was rolled on a roller mixer for about 24 hours. The solution was centrifuged at 2000 rpm for 3 minutes using a Megafuge 1.0 centrifuge, commercially available from Heraeus Holding GmbH, Germany. An Ubbelohde viscosity measurement according to DIN 51562-1:1999-01 (January 1999) was carried out. The measurement was done at 20 °C.

Content of ether and ester groups of (HPMCAS)

The content of ether groups in the esterified cellulose ether was determined in the same manner as described for "Hypromellose", United States Pharmacopeia and National Formulary, USP 35, pp 3467-3469.

The ester substitution with acetyl groups ($-CO-CH_3$) and the ester substitution with succinoyl groups ($-CO-CH_2-CH_2-COOH$) were determined according to Hypromellose Acetate Succinate, United States Pharmacopia and National Formulary, NF 29, pp. 1548-1550". Reported values for ester substitution were corrected for volatiles (determined as described in section "loss on drying" in the above HPMCAS monograph).

Determination of $M_w$ and $M_n$ of HPMCAS

Mw and Mn were measured according to Journal of Pharmaceutical and Biomedical Analysis 56 (2011) 743 unless stated otherwise. The mobile phase was a mixture of 40 parts by volume of acetonitrile and 60 parts by volume of aqueous buffer containing 50 mM NaH2PO4 and 0.1 M NaNO3. The mobile phase was adjusted to a pH of 8.0.

Solutions of the cellulose ether esters were filtered into a HPLC vial through a syringe filter of 0.45 µm pore size.

More specifically, the utilized Chemicals and solvents were:

Polyethylene oxide standard materials (abbreviated as PEOX 20 K and PEOX 30 K) were purchased from Agilent Technologies, Inc. Palo Alto, CA, catalog number PL2083-1005 and PL2083-2005.

Acetonitrile (HPLC grade ≥ 99.9 %, CHROMASOL plus), catalog number 34998, sodium hydroxide (semiconductor grade, 99.99 %, trace metal base), catalog number 306576, water (HPLC grade, CHROMASOLV Plus) catalog number 34877 and sodium nitrate (99,995 %, trace metal base) catalog number 229938 were purchased from Sigma-Aldrich, Switzerland.

Sodium dihydrogen phosphate (≥ 99.999 % TraceSelect) catalog number 71492 was purchased from FLUKA, Switzerland.

The normalization solution of PEOX20 K at 5 mg/mL, the standard solution of PEOX30 K at 2 mg/mL, and the sample solution of HPMCAS at 2 mg/mL were prepared by adding a weighed amount of polymer into a vial and dissolving it with a measured volume of mobile phase. All solutions were allowed to dissolve at room temperature in the capped vial for 24 h with stirring using a PTFE-coated magnetic stirring bar.

The normalization solution (PEOX 20k, single preparation, N) and the standard solution (PEOX30 K, double preparation, S1 and S2) were filtered into a HPLC vial through a syringe filter of 0.02 µm pore size and 25 mm diameter (Whatman Anatop 25, catalog number 6809-2002), Whatman.

The test sample solution (HPMCAS, prepared in duplicate, T1, T2) and a laboratory standard (HPMCAS, single preparation, LS) were filtered into a HPLC vial through a syringe filter of 0.45 µm pore size (Nylon, e.g. Acrodisc 13 mm VWR catalog number 514-4010).

Chromatographic condition and run sequence were conducted as described by Chen, R. et al.; Journal of Pharmaceutical and Biomedical Analysis 56 (2011) 743- 748). The SEC-MALLS instrument set-up included a HP1100 HPLC system from Agilent Technologies, Inc. Palo Alto, CA; a DAWN Heleos II 18 angle laser light scattering detector and a OPTILAB rex refractive index detector, both from Wyatt Technologies, Inc. Santa Barbara, CA. The analytical size exclusion column (TSK-GEL® GMPWXL, 300 × 7.8 mm) was purchased from Tosoh Bioscience. Both the OPTILAB and the DAWN were operated at 35 °C. The analytical SEC column was operated at room temperature (24 ± 5 °C). The mobile phase was a mixture of 40 volume parts of acetonitrile and 60 volume parts of aqueous buffer containing 50 mM NaH2PO4 and 0.1 M NaNO3 prepared as follows:

Aqueous buffer: 7.20 g of sodium dihydrogen phosphate and 10.2 g of sodium nitrate were added to 1.2 L purified water in a clean 2 L glass bottle under stirring until dissolution.

Mobile phase: 800 mL of acetonitrile were added to 1.2 L of the aqueous buffer prepared above, and stirred until a good mixture was achieved and the temperature equilibrated to ambient temperature.

The mobile phase was pH adjusted to 8.0 with 10M NaOH and filtered through a 0.2 m nylon membrane filter. The flow rate was 0.5 mL/min with in-line degassing. The injection volume was 100 µL and the analysis time was 35 min.

The MALLS data were collected and processed by Wyatt ASTRA software (version 5.3.4.20) using dn/dc value (refractive index increment) of 0.120 mL/g for HPMCAS. The light scattering signals of detector Nos. 1-4, 17, and 18) were not used in the molecular weight calculation. A representative chromatographic run sequence is given below: B, N, LS, S1 (5x), S2, T1 (2x), T2 (2x), T3 (2x), T4 (2x), S2, T5(2x), etc., S2, LS, W, where, B represents blank injection of mobile phase, N1 represents normalization solution; LS represents a laboratory standard HPMCAS; S1 and S2 represent standard solutions one and two, respectively; T1, T2, T3, T4, and T5 represent test sample solutions and W represents water injection. (2x) and (5x) denote the number of injections of the same solution.

Both the OPTILAB and the DAWN were calibrated periodically according to the manufacturer's recommended procedures and frequency. A 100 µL injection of a 5 mg/mL polyethylene oxide standard (PEOX20 K) was employed for normalizing all angle light scattering detectors relative to 90° detector for each run sequence.

Use of this mono-dispersed polymer standard also enabled the volume delay between the OPTILAB and the DAWN to be determined, permitting proper alignment of the light scattering signals to the refractive index signal. This is necessary for the calculation of the weight-averaged molecular weight (Mw) for each data slice.

Comparative Example A

A hydroxypropyl methylcellulose (HPMC), glacial acetic acid and a first portion of sodium acetate were introduced into a reaction vessel at the amounts listed in Table 1 below. The amount of HPMC was calculated on a dried basis. The HPMC had a methoxyl substitution ($DS_M$) and a hydroxypropoxyl substitution ($MS_{HP}$) as listed in Table 2 below and a viscosity of about 3 mPa·s, measured as a 2 weight-% aqueous solution at 20 °C according to ASTM D2363 - 79 (Reapproved 2006). The HPMC is commercially available from The Dow Chemical Company as Methocel E3 LV Premium cellulose ether. The mixture of sodium acetate, HPMC and acetic acid was heated to 85°C and kept at this temperature for 15 minutes under stirring. Afterwards succinic anhydride and three minutes later acetic anhydride were added to the reaction vessel at the amounts listed in Table 1 below. The resulting reaction mixture was kept at 85°C for 30 minutes. Subsequently a second portion of sodium acetate at the amount listed in Table 1 was introduced into the reaction vessel under stirring. After the addition of the second portion of sodium acetate, the reaction mixture was allowed to react for 4 hours. This time period is listed as total reaction time in Table 1 below.

The product was removed from the reactor, precipitated in 3 L of water and washed with water having a temperature of 21 °C by applying high shear mixing using an Ultra-Turrax stirrer S50-G45 running at 5200 rpm. Washing was conducted in several portions with intermediate filtration steps to obtain HPMCAS of high purity. After the last filtration step the product was dried at 55°C overnight.

Example 1

The procedure of Comparative Example A was repeated, except that 2.5 hours after the addition of the second portion of sodium acetate an additional amount of acetic acid was added to the reaction mixture at the amount listed in Table 1 below. After the addition of the second amount of acetic acid the reaction mixture was allowed to react for additional 1.5 hours.

Examples 2 and 3

The procedure of Comparative Example A was repeated, except that 2 hours after the addition of the second portion of sodium acetate an additional amount of acetic acid was added to the reaction mixture at the amounts listed in Table 1 below. After the addition of the second amount of acetic acid the reaction mixture was allowed to react for additional 2 hours.

Example 4

The procedure of Comparative Example A was repeated, except that 1.5 hours after the addition of the second portion of sodium acetate an additional amount of acetic acid was added to the reaction mixture at the amount listed in Table 1 below. After the addition of the second amount of acetic acid the reaction mixture was allowed to react for additional 2.5 hours.

The properties of the HPMCAS produced according to the processes of Comparative Example A and of Examples 1-4 are listed in Table 2 below. In Table 2 the abbreviations have the following meanings:

$DS_M$ = DS(methoxyl): degree of substitution with methoxyl groups;
$MS_{HP}$ = MS(hydroxypropoxyl): molar substitution with hydroxypropoxyl groups;
$DS_{Ac}$: degree of substitution with acetyl groups;
$DS_s$: degree of substitution with succinoyl groups;

Table 1

| (Comp.) Example | HPMC | | acetic acid in stage i) | | Sodium acetate, 1st portion | | Succinic anhydride | | Acetic anhydride | | Sodium acetate, 2nd portion | | Total Reaction time* | Amount / time of acetic acid addition in stage ii)* |
|---|---|---|---|---|---|---|---|---|---|---|---|---|---|---|
| | g | mol | g | mol/mol HPMC | g | mol/mol HPMC | g | mol/mol HPMC | g | mol/mol HPMC | g | mol/mol HPMC | | |
| A | 230 | 1.14 | 438 | 6.4 | 41.44 | 0.64 | 91.0 | 0.80 | 286 | 2.57 | 124.31 | 1.93 | 4 h | 0 |
| 1 | 230 | 1.14 | 438 | 6.4 | 41.44 | 0.64 | 91.0 | 0.80 | 286 | 2.57 | 124.31 | 1.93 | 4 h | 100 g / 2.5h |
| 2 | 230 | 1.14 | 438 | 6.4 | 41.44 | 0.64 | 91.0 | 0.80 | 286 | 2.57 | 124.31 | 1.93 | 4 h | 200 g / 2h |
| 3 | 230 | 1.14 | 438 | 6.4 | 41.44 | 0.64 | 91.0 | 0.80 | 286 | 2.57 | 124.31 | 1.93 | 4 h | 250 g / 2h |
| 4 | 230 | 1.14 | 438 | 6.4 | 41.44 | 0.64 | 91.0 | 0.80 | 286 | 2.57 | 124.31 | 1.93 | 4 h | 100 g / 1.5h |
| * Time counted from 2nd addition of sodium acetate | | | | | | | | | | | | | | |

Table 2

| (Comparative) Example | Molecular weight (kDA) | | 10% viscosity in acetone (mPa·s) | Methoxyl (%) | Hydroxy-propoxyl (%) | Acetyl (%) | Succinoyl (%) | $DS_M$ | $MS_{HP}$ | $DS_{Ac}$ | $DS_S$ |
|---|---|---|---|---|---|---|---|---|---|---|---|
| | Mn | Mw | | | | | | | | | |
| A | 156 | 460 | 95 | 21.5 | 6.9 | 8.8 | 17.1 | 1.89 | 0.25 | 0.56 | 0.46 |
| 1 | 139 | 423 | 80 | 21.7 | 6.8 | 9.0 | 17.2 | 1.92 | 0.25 | 0.57 | 0.47 |
| 2 | 165 | 464 | 75 | 21.9 | 6.9 | 8.8 | 17.1 | 1.93 | 0.25 | 0.56 | 0.46 |
| 3 | 150 | 409 | 63 | 22.0 | 6.9 | 8.8 | 17.4 | 1.95 | 0.25 | 0.56 | 0.47 |
| 4 | 144 | 402 | 60 | 21.8 | 6.8 | 9.2 | 17.9 | 1.96 | 0.25 | 0.60 | 0.49 |

The results in Table 2 above illustrate that by the process of the present invention esterified cellulose ethers are produced which have a surprisingly high weight average molecular weight $M_w$ at a surprisingly low viscosity in acetone.

The HPMCAS produced according to Example 1 has a weight average molecular weight $M_w$ which is 8 % lower than that of Comparative Example A, but the viscosity of the HPMCAS of Example 1 is even 16 % lower than that of Comparative Example A.

The HPMCAS produced according to Example 2 has a weight average molecular weight $M_w$ which is about the same as that of Comparative Example A, but the viscosity of the HPMCAS of Example 2 is even 21 % lower than that of Comparative Example A.

The HPMCAS produced according to Example 3 has a weight average molecular weight $M_w$ which is 11 % lower than that of Comparative Example A, but the viscosity of the HPMCAS of Example 3 is even 34 % lower than that of Comparative Example A.

The HPMCAS produced according to Example 4 has a weight average molecular weight $M_w$ which is 13 % lower than that of Comparative Example A, but the viscosity of the HPMCAS of Example 1 is even 37 % lower than that of Comparative Example A.

**Claims**

1.    A process for producing an esterified cellulose ether comprising the stages of

i) preparing a reaction mixture comprising a cellulose ether, an aliphatic monocarboxylic acid anhydride, a dicarboxylic acid anhydride, and a reaction diluent and heating the reaction mixture to a temperature of from 60°C to 110 °C prior to, during or after mixing the components of the reaction mixture to conduct an esterification reaction, wherein an aliphatic carboxylic acid is used as a reaction diluent and wherein the reaction mixture additionally comprises an alkali metal carboxylate as a reaction catalyst, and
ii) before the esterification reaction is completed, adding an additional amount of reaction diluent continuously or in one or more portions to the reaction mixture and allowing the esterification reaction to further proceed, wherein the cellulose ether is an alkyl cellulose, a hydroxyalkylcellulose or a hydroxyalkyl alkylcellulose, the aliphatic monocarboxylic acid anhydride is acetic anhydride, butyric anhydride or propionic anhydride and the dicarboxylic acid anhydride is succinic anhydride, maleic anhydride or phthalic anhydride.

2.    The process of claim 1 the molar ratio [aliphatic carboxylic acid / anhydroglucose units of cellulose ether] in stage i) is from [4.0 / 1.0] to [11.5 / 1.0].

3.    The process of claim 2 wherein the aliphatic carboxylic acid is added in stage ii) at a molar ratio [aliphatic carboxylic acid / anhydroglucose units of cellulose ether] of from [0.5 / 1.0] to [7.0/1.0].

4.    The process of any one of claims 1 to 3 wherein an additional amount of reaction diluent is added to the reaction mixture when more than 20% of the entire reaction time has passed.

5.    The process of any one of claims 1 to 4 wherein an additional amount of reaction diluent is added to the reaction mixture when less than 80% of the entire reaction time has passed.

6.    The process of any one of claims 1 to 5 wherein from 50 to 90 percent of the reaction diluent is added in stage i) and from 10 to 50 percent of the reaction diluent is added in stage ii), based on the total weight of the reaction diluent in the process.

7.    The process of any one of claims 1 to 6 wherein stage i) of the process comprises the steps of

iA) dissolving or dispersing a cellulose ether and a first amount of a reaction catalyst in a reaction diluent,
iB) heating the obtained mixture to a temperature of 60 °C to 110 °C before, during or after adding an aliphatic monocarboxylic acid anhydride and a dicarboxylic acid anhydride to the mixture obtained in step iA), and
iC) adding a second amount of a reaction catalyst.

8.    The process of claim 7 wherein the first amount of reaction catalyst added in step iA) is 15 to 35 percent and the second amount of reaction catalyst added in step iC) is 65 to 85 percent, based on the total amount of reaction catalyst added in stage i) of the process.

**9.** The process of any one of claims 1 to 8 wherein hydroxypropyl methylcellulose is esterified with succinic anhydride and acetic anhydride to produce hydroxypropyl methyl cellulose acetate succinate.

**10.** The process of any one of claims 1 to 9 wherein the produced esterified cellulose ether has a weight average molecular weight $M_w$ of from 100,000 to 500,000 Dalton.

**11.** The process of any one of claims 1 to 10 wherein the produced esterified cellulose ether has a viscosity of up to 90 mPa·s, measured as a 10 wt.-% solution of the esterified cellulose ether in acetone at 20 °C.

**Patentansprüche**

**1.** Ein Verfahren zum Herstellen eines veresterten Celluloseethers umfassend die Schritte des:

i) Herstellens einer Reaktionsmischung umfassend einen Celluloseether, ein aliphatisches Monocarbonsäureanhydrid, ein Dicarbonsäureanhydrid und ein Reaktionsverdünnungsmittel und Erwärmens der Reaktionsmischung auf eine Temperatur von 60 °C bis 110 °C vor, während oder nach dem Vermischen der Bestandteile der Reaktionsmischung, um eine Veresterungsreaktion durchzuführen, wobei eine aliphatische Carbonsäure als ein Reaktionsverdünnungsmittel verwendet wird und wobei die Reaktionsmischung des Weiteren ein Alkalimetallcarbonsäuresalz als einen Reaktionskatalysator enthält und
ii) bevor die Veresterungsreaktion beendet ist, Hinzugebens einer zusätzlichen Menge von Reaktionsverdünnungsmittel in kontinuierlicher Weise oder in einem oder mehreren Teil(en) zu der Reaktionsmischung und Voranschreitenlassens der Veresterungsreaktion, wobei der Celluloseether eine Alkylcellulose, eine Hydroxyalkylcellulose oder eine Hydroxyalkylalkylcellulose ist, das aliphatische Monocarbonsäureanhydrid Essigsäureanhydrid, Buttersäureanhydrid oder Propionsäureanhydrid ist und das Dicarbonsäureanhydrid Bernsteinsäureanhydrid, Maleinsäureanhydrid oder Phthalsäureanhydrid ist.

**2.** Das Verfahren gemäß Anspruch 1, wobei das molare Verhältnis [aliphatische Carbonsäure/Anhydroglucose-Einheiten von Celluloseether] in Schritt i) von [4,0/1,0] bis [11,5/1,0] beträgt.

**3.** Das Verfahren gemäß Anspruch 2, wobei die aliphatische Carbonsäure in Schritt ii) bei einem molaren Verhältnis [aliphatische Carbonsäure/Anhydroglucose-Einheiten von Celluloseether] von [0,5/1,0] bis 7,0/1,0] hinzugegeben wird.

**4.** Das Verfahren gemäß einem der Ansprüche 1 bis 3, wobei eine zusätzliche Menge von Reaktionsverdünnungsmittel zu der Reaktionsmischung hinzugegeben wird, wenn mehr als 20 % der gesamten Reaktionszeit verstrichen sind.

**5.** Das Verfahren gemäß einem der Ansprüche 1 bis 4, wobei eine zusätzliche Menge an Reaktionsverdünnungsmittel zu der Reaktionsmischung hinzugegeben wird, wenn weniger als 80 % der gesamten Reaktionszeit verstrichen sind.

**6.** Das Verfahren gemäß einem der Ansprüche 1 bis 5, wobei von 50 bis 90 % des Reaktionsverdünnungsmittels in Stufe i) hinzugegeben werden und von 10 bis 50 % des Reaktionsverdünnungsmittels in Stufe ii) hinzugegeben werden, bezogen auf das Gesamtgewicht des Reaktionsverdünnungsmittels in dem Verfahren.

**7.** Das Verfahren gemäß einem der Ansprüche 1 bis 6, wobei Stufe i) des Verfahrens die Schritte des

iA) Lösens oder Dispergierens eines Celluloseethers und einer ersten Menge von einem Reaktionskatalysator in einem Reaktionsverdünnungsmittel,
iB) Erwärmens der erhaltenen Mischung auf eine Temperatur von 60 °C bis 110 °C vor, während oder nach Hinzugeben eines aliphatischen Monocarbonsäureanhydrids und eines Dicarbonsäureanhydrids zu der in Schritt iA) erhaltenen Mischung und
iC) Hinzugebens einer zweiten Menge eines Reaktionskatalysators umfasst.

**8.** Das Verfahren gemäß Anspruch 7, wobei die erste Menge von Reaktionskatalysator, die in Schritt iA) hinzugegeben wird, 15 bis 35 % beträgt und die zweite Menge von Reaktionskatalysator, die in Schritt iC) hinzugegeben wird, 65 bis 85 % beträgt, bezogen auf die gesamte Menge von Reaktionskatalysator, die in Stufe i) des Verfahrens hinzugegeben wird.

9. Das Verfahren gemäß einem der Ansprüche 1 bis 8, wobei Hydroxypropylmethylcellulose mit Bernsteinsäureanhydrid und Essigsäureanhydrid verestert wird, um Hydroxypropylmethylcellulose-Acetat-Succinat herzustellen.

10. Das Verfahren gemäß einem der Ansprüche 1 bis 9, wobei der hergestellte veresterte Celluloseether ein gewichtsmittleres Molekulargewicht $M_w$ von 100.000 bis 500.000 Dalton aufweist.

11. Das Verfahren gemäß einem der Ansprüche 1 bis 10, wobei der hergestellte veresterte Celluloseether eine Viskosität von bis zu 90 mPa·s aufweist, gemessen als eine 10 gewichtsprozentige Lösung des veresterten Celluloseethers in Aceton bei 20 °C.

**Revendications**

1. Procédé de production d'un éther de cellulose estérifié, qui comporte les stades suivants :

    i) préparer un mélange réactionnel comprenant un éther de cellulose, un anhydride d'acide monocarboxylique aliphatique, un anhydride d'acide dicarboxylique et un diluant de réaction, et chauffer ce mélange réactionnel à une température de 60 à 110 °C avant, pendant ou après le mélangeage des composants du mélange réactionnel afin de conduire une réaction d'estérification, étant entendu qu'un acide carboxylique aliphatique est utilisé en tant que diluant de réaction et que le mélange réactionnel comprend en plus, en tant que catalyseur de réaction, un carboxylate de métal alcalin,
    ii) et ajouter au mélange réactionnel, avant que s'achève la réaction d'estérification, un supplément de diluant de réaction, en continu ou en une ou plusieurs portions, et laisser la réaction d'estérification se poursuivre,

    et dans lequel procédé

    - l'éther de cellulose est une alkyl-cellulose, une hydroxyalkylcellulose ou une hydroxyalkyl-alkyl-cellulose,
    - l'anhydride d'acide monocarboxylique aliphatique est de l'anhydride acétique, de l'anhydride butyrique ou de l'anhydride propionique,
    - et l'anhydride d'acide dicarboxylique est de l'anhydride succinique, de l'anhydride maléique ou de l'anhydride phtalique.

2. Procédé conforme à la revendication 1, dans lequel, au stade (i), le rapport molaire de l'acide carboxylique aliphatique aux motifs d'anhydro-glucose de l'éther de cellulose vaut de 4,0/1,0 à 11,5/1,0.

3. Procédé conforme à la revendication 2, dans lequel, au stade (ii), l'acide carboxylique aliphatique est ajouté en un rapport molaire de l'acide carboxylique aliphatique aux motifs d'anhydro-glucose de l'éther de cellulose valant de 0,5/1,0 à 7,0/1,0.

4. Procédé conforme à l'une des revendications 1 à 3, dans lequel on ajoute un supplément du diluant de réaction au mélange réactionnel quand il s'est écoulé plus de 20 % de la durée totale de la réaction.

5. Procédé conforme à l'une des revendications 1 à 4, dans lequel on ajoute un supplément du diluant de réaction au mélange réactionnel quand il s'est écoulé moins de 80 % de la durée totale de la réaction.

6. Procédé conforme à l'une des revendications 1 à 5, dans lequel, par rapport au poids total du diluant de réaction utilisé dans le procédé, de 50 à 90 % du diluant de réaction sont ajoutés au stade (i) et de 10 à 50 % du diluant de réaction sont ajoutés au stade (ii).

7. Procédé conforme à l'une des revendications 1 à 6, dans lequel le stade (i) du procédé comporte les étapes suivantes :

    iA) dissoudre ou disperser, dans un diluant de réaction, un éther de cellulose et une première quantité d'un catalyseur de réaction,
    iB) chauffer le mélange ainsi obtenu à une température de 60 à 110 °C avant, pendant ou après l'addition d'un anhydride d'acide monocarboxylique aliphatique et d'un anhydride d'acide dicarboxylique au mélange obtenu dans l'étape (iA),
    iC) et ajouter une deuxième quantité d'un catalyseur de réaction.

**8.** Procédé conforme à la revendication 7, dans lequel, par rapport à la quantité totale de catalyseur de réaction ajoutée au stade (i) du procédé, la première quantité de catalyseur de réaction ajoutée lors de l'étape (iA) en représente de 15 à 35 % et la deuxième quantité de catalyseur de réaction ajoutée lors de l'étape (iC) en représente de 65 à 85 %.

**9.** Procédé conforme à l'une des revendications 1 à 8, dans lequel de l'hydroxypropyl-méthyl-cellulose est estérifiée au moyen d'anhydride succinique et d'anhydride acétique, pour donner un acéto-succinate d'hydroxypropyl-méthyl-cellulose.

**10.** Procédé conforme à l'une des revendications 1 à 9, dans lequel l'éther de cellulose estérifié produit présente une masse molaire moyenne en poids $M_w$ de 100 000 à 500 000 daltons.

**11.** Procédé conforme à l'une des revendications 1 à 10, dans lequel l'éther de cellulose estérifié produit présente une viscosité, mesurée dans une solution à 10 % en poids de l'éther de cellulose estérifié dans de l'acétone, à 20 °C, vaut jusqu'à 90 mPa.s.

REFERENCES CITED IN THE DESCRIPTION

**Patent documents cited in the description**

- US 4226981 A
- US 4365060 A
- EP 0219426 A
- WO 2005115330 A
- WO 2011159626 A
- WO 2014133885 A
- WO 2014031447 A
- WO 2014031448 A

- WO 2014137779 A
- EP 1141029 A
- EP 0210917 A
- EP 1423433 A
- US 4316982 A
- US 13030394 W
- WO 2013148154 A

**Non-patent literature cited in the description**

- **EDGAR et al.** *Cellulose,* 2007, vol. 14, 49-64
- **G. BARTELMUS ; R. KETTERER.** *Z. Anal. Chem.,* 1977, vol. 286, 161-190
- Hypromellose Acetate Succinate. *United States Pharmacopia and National Formulary,* vol. 29, 1548-1550
- Hypromellose. *United States Pharmacopeia and National Formulary, USP,* vol. 35, 3467-3469

- *Journal of Pharmaceutical and Biomedical Analysis,* 2011, vol. 56, 743
- Hypromellose Acetate Succinate. *United States Pharmacopia and National Formulary, NF,* vol. 29, 1548-1550
- **CHEN, R. et al.** *Journal of Pharmaceutical and Biomedical Analysis,* 2011, vol. 56, 743-748